Europäisches Patentamt

(19) European Patent Office

Office européen des brevets

(11) Publication number: **0 259 953 B1**

# EUROPEAN PATENT SPECIFICATION

(45) Date of publication of patent specification: **06.04.94**   (51) Int. Cl.5: **C12N 15/12**, C07K 13/00

(21) Application number: **87306066.9**

(22) Date of filing: **09.07.87**

(54) **Cloning and expression of acidic fibroblast growth factor.**

(30) Priority: **11.07.86 US 884460**
          **04.06.87 US 54991**

(43) Date of publication of application:
   **16.03.88 Bulletin 88/11**

(45) Publication of the grant of the patent:
   **06.04.94 Bulletin 94/14**

(84) Designated Contracting States:
   **AT BE CH DE ES FR GB GR IT LI LU NL SE**

(56) References cited:
   **EP-A- 0 131 150**
   **EP-A- 0 225 701**
   **WO-A-87/05332**

   **Biochem. Biophys. Res. Commun., vol. 135 (1986), pages 541-548**

   **SCIENCE, vol.230, no. 4732, 1985, pp. 1385-1388; USA G. GIMENEZ-GALLEGO et al.:"Brain-derived acidic fibroblast growth factor: Complete amino acid sequence and homologies."**

(73) Proprietor: **MERCK & CO. INC.**
   **126, East Lincoln Avenue**
   **P.O. Box 2000**
   **Rahway New Jersey 07065-0900(US)**

(72) Inventor: **Linemeyer, David L.**
   **526 Clark Street**
   **Westfield New Jersey 07090(US)**
   Inventor: **Kelly, Linda J.**
   **219 Bronx River Road Apt. 41**
   **Yonkers New York 10704(US)**
   Inventor: **Thomas, Kenneth A., Jr.**
   **245 Washington Avenue**
   **Chatham New Jersey 07928(US)**
   Inventor: **Gimenez-Gallego, Guillermo**
   **2652 Kennedy Boulevard**
   **Jersey City New Jersey 07306(US)**

(74) Representative: **Cole, William Gwyn**
   **European Patent Department**
   **Merck & Co., Inc.**
   **Terlings Park**
   **Eastwick Road**
   **Harlow Essex CM20 2OR (GB)**

CHEMICAL ABSTACTS, vol. 105, no. 19, November 10,1985, page 89, ref. no. 165184n; Columbus, Ohio, US G. GIMENEZ-GALLEGO et al.:"The complete amino acid sequence of human brain-derived acidic fibroblast growth factor." & BIOCHEM. BIOPHYS. RES. COMMUN,1986, vol. 138,no. 2, pp. 611-617n(Cat. D)

BIOLOGICAL ABSTRACTS/RRM, vol. 33, 1987, ref. no. 40030; Philadelphia, Pa. US J. ABRA-HAM et al.:"Cloning and characterization of the genes for the angiogenic proteins basic and acidic fibroblast growth factor ," & J. CELL BIOCHEMSUPPL., 1987, vol. 0, no. 11, part A, page 50

BIO/TECHNOGY, vol. 5, no. 9, September 1987, pp. 960-965; London, GB D.L. LINEMEYER et al.: "Expression in Escherichia coli of a chemically synthe- sized gene for biologically active bovine acidic fibroblast growth factor

SCIENCE, vol. 233, no. 4763, August 1, 1986, pp.541-545; USA M. JAYE et al.:

Biological Abstracts/RRM, vol. 31, ref. no. 46304, 1986, Philadelphia, Pa.,US J.C. Fiddes et al.: "Isolation and characterization of clones encoding basic and acidic fibroblast growth factors." & Cell Biochem, 1986, vol. 0, n.10. part C, page 149

Chemical Abstracts, vol. 104, no. 21, May 26, 1986, page 118, ref. no. 180694b; Columbus, Ohio, US, G. Gimenez-Gallego et al.: "Human brain-derived acidic and basic fibroblast growth factors: amino terminal sequences and specific mitogenic activities." & Biochem. Biophys. Res. Commun., 1986, vol 135, no. 2, pages 541-548

**Description**

BRIEF DESCRIPTION OF THE DRAWING

Figure I is a diagram of the pKK223-3 plasmid containing the gene for aFGF.

BACKGROUND OF THE INVENTION

Brain derived fibroblast mitogens were first described by Trowell et al., J. Exp. Biol. 16: 60-70 (1939) and Hoffman, Growth 4: 361-376 (1940). It was subsequently shown that pituitary extracts also had potent mitogenic activity for fibroblasts, Armelin, Proc. Natl. Acad. Sci USA 70: 2702-2706 (1973). Partial purification of both brain and pituitary fibroblast growth factor (FGF) revealed mitogenic activity against a variety of types of differentiated cells including vascular endothelial cells, Gospodarowicz et al., Natl. Cancer Inst. Monogr. 48: 109-130 (1978). It has recently been shown that FGF exists in two forms, acidic FGF (aFGF) and basic FGF (bFGF), and both forms have been identified in brain preparations, Thomas and Gimenez-Gallego, TIBS 11: 81-84 (1986). Numerous cell types respond to stimulation with either purified aFGF or bFGF to synthesize DNA and divide, including primary fibroblasts, vascular and corneal endothelial cells, chondrocytes, osteoblasts, myoblasts, smooth muscle and glial cells, Esch et al., Proc. Natl. Acad. Sci. USA 82: 6507-6511 (1985); Kuo et al., Fed. Proc. 44: 695 (1985).

Pure bovine brain-derived aFGF not only acts as a potent mitogen for vascular endothelial cells in culture but also induces blood vessel growth in vivo, Thomas et al., Proc. Natl. Acad. Sci. USA 82: 6409-6413 (1985). The fibroblast mitogenic activity of aFGF can also be utilized to promote wound healing, Thomas, U.S. Patent 4,444,760. The present invention provides a genetic construct and means of expression that allows the production of large amounts of pure aFGF that can be used therapeutically.

OBJECTS OF THE INVENTION

It is, accordingly, an object of the present invention to provide a nucleotide base sequence for both bovine aFGF and human aFGF from the amino acid sequences of the specific proteins. Another object is to produce genes coding for the specific aFGFs and incorporate the genes into appropriate cloning vectors A further object is to transform an appropriate host with each of the recombinant vectors and to induce expression of the specific aFGF genes. Another object is to isolate and purify biologically active bovine aFGF and human aFGF. These and other objects of the present invention will be apparent from the following description.

SUMMARY OF THE INVENTION

Unique genes coding for the amino acid sequence of bovine acidic fibroblast growth factor (aFGF) and human aFGF are constructed. The bovine gene is derived from reverse translation of the aFGF amino acid sequence while the human gene is derived by specific point mutations of the bovine gene. Each gene construct is inserted into an expression vector which is used to transform an appropriate host. The transformed host cells produce recombinant aFGF (r-aFGF), human or bovine, which is purified and has an activity equivalent to the native protein.

DETAILED DESCRIPTION

Acidic fibroblast growth factor exists in various microheterogeneous forms which are isolated from the various tissue sources and cell types known to contain aFGF. Microheterogeneous forms as used herein refers to a single gene product, that is a peptide produced from a single gene unit of DNA, which is structurally modified following translation. The structural modifications, however, do not result in any significant alterations of biological activity of the peptide. The modifications may take place either in vivo or during the isolation and purification process. In vivo modification results in but is not limited to proteolysis, glycosylation, phosphorylation or acetylation at the N-terminus. Proteolysis may include exoproteolysis wherein one or more terminal amino acids are sequentially, enzymatically cleaved to produce a micro-heterogeneous form which has fewer amino acids than the original gene product. Endoproteolytic modification results from the action of endoproteases which cleave the peptide at specific locations within the amino acid sequence. Similar modifications can occur during the purification process which also results in production of micro- heterogeneous forms. The most common modification occuring during purification is proteolysis which is generally held to a minimum by the use of protease inhibitors. Under most conditions a mixture of microheterogeneous forms are present following purification of native aFGF. Native aFGF refers to aFGF isolated and purified from tissues or cells that contain aFGF.

The invention is contemplated to include all mammalian microheterogeneous forms of acidic fibroblast growth factor. The preferred embodiments include bovine and human microheterogeneous forms of aFGF. The most perferred microheterogeneous forms of bovine aFGF include a 154 amino acid form, a 140 amino acid form and a 134 amino acid form. The 140 amino acid form is shown in TABLE III and is the most preferred of the bovine species. The 154 amino acid form includes the following additional amino acids;

```
Ala-Glu-Gly-Glu-Thr-Thr-Thr-Phe-Thr-Ala-Leu-Thr-Glu-
Lys,
```

with the carboxyl terminus Lys attached to the amino terminus Phe at the first position of the 140 amino acid form. The 134 amino acid form is identical to the 140 amino acid form except that the first 6 amino acids of the amino terminus have been removed. When isolated the relative amounts of these microheterogeneous forms vary depending on the process used but all preparations contain at least a portion of each form.

Human aFGF exhibits a similar microheterogeneity to that of bovine aFGF. The most preferred microheterogeneous forms of human aFGF include a 154 amino acid form, a 140 amino acid form and a 139 amino acid form. The human 140 amino acid form differs from the bovine form by eleven amino acids, as shown in TABLE V. The 154 amino acid form contains the exact sequence of the human 140 amino acid form plus the 14 additional amino acids associated with the bovine 154 amino acid form, with one exception. The amino acid at the fifth position of the N-terminus or at the -10 position as determined from the 140 amino acid Phe n-terminus in the human form is isoleucine and is substituted for the threonine in the bovine form. The additional 14 amino acid human N-terminal sequence is;

```
Ala-Glu-Gly-Glu-Ile-Thr-Thr-Phe-Thr-
Ala-Lue-Thr-Glu-Lys.
```

A third form of human aFGF contains 139 amino acids and is equivalent to the human 140 amino acid form with the amino terminus phenylalanine removed. The amino terminus asparagine residue may be deamidated to aspartic acid in the 139 amino acid form of human aFGF. The 140 and 139 amino acid forms are the most preferred forms of the human microheterogeneous forms.

Mammalian r-aFGF is produced by cloning the natural gene from either the genomic DNA or cDNA, or by construction of a gene for one of the microheterogeneous forms of the protein based on the known amino acid sequences of these microheterogeneous forms of aFGF from mammalian species including man. Genomic DNA is extracted from mammalian brain or pituitary cells and prepared for cloning by either random fragmentation of high-molecular-weight DNA following the technique of Maniatis et al., Cell 15: 687-701 (1978) or by cleavage with a restriction enzyme by the method of Smithies et al., Science 202: 1284-1289 (1978). The genomic DNA is then incorporated into an appropriate cloning vector, generally E. coli lambda phage, see Maniatis et al., Molecular Cloning, A Laboratory Manual, Cold Spring Harbor Laboratory, Cold Spring Harbor, New York (1982).

To obtain cDNA for aFGF, poly (A)-containing RNA is extracted from cells that express aFGF by the method of Aviv and Leder, Proc. Natl. Acad. Sci. 69: 1408-1412 (1972). The cDNA is prepared using reverse transcriptase and DNA polymerase using standard techniques, as described in Maniatis et al., Molecular Cloning, a Laboratory Manual, Cold Spring Harbor Laboratory, Cold Spring Harbor, New York (1982). The cDNA is tailed and cloned into an appropriate vector, usually pBR322, by a technique similar to that of Wensink, et al., Cell 3: 315-325 (1974).

The clonal genomic DNA or cDNA libraries are screened to identify the clones containing aFGF sequences by hybridization with an oligonucleotide probe. The sequence of the oligonucleotide hybridization probe is based on the determined amino acid sequence of aFGF. Maniatis et al. supra, Anderson and Kingston, Proc. Natl. Acad. Sci. USA 80:6838-6842 (1983) and Suggs et al., Proc. Natl. Acad. Sci. USA 78:6613-6617 (1981) describe various procedures for screening genomic and cDNA clones.

The preferred procedure for obtaining a gene for mammalian aFGF is to synthesize the gene. The gene may be synthesized based on the amino acid sequence of a microheterogeneous form of aFGF obtained from any mammal including man. The preferred method is to use the bovine amino acid sequence for aFGF and chemically point mutate the base sequence to produce the genes for other species. The amino acid sequences for bovine and human aFGF are disclosed in U.S. Patent Application Serial No. 868,473 filed May 30, 1986 which is a continuation-in-part of U.S. Patent Application Serial No. 774,359 filed September 12, 1985 which is a continuation-in-part of U.S. Patent Application Serial No. 685,923, filed December 24, 1984 (now abandoned).

The synthetic genes are based on the determined bovine amino acid sequence subsequently described by Gimenez-Gallego et al., Science 230: 1385-1388 (1985) and the human amino acid sequence as described by Gimenez-Gallego et al. Biochem. Biophys. Res. Comm., 138: 611-617 (1986). The unique nucleotide sequence of the 140 amino acid form of bovine aFGF is derived from reverse translation of the amino acid sequence by a technique similar to that of Itakura et al., Science 198: 1056-1063 (1977). The various novel nucleotide sequences corresponding to the native amino acid sequence of bovine aFGF are shown in the following table:

## TABLE I

| | | | | | 5 | | | | | 10 | | | | | 15 | | | | | 20 |
|---|---|---|---|---|---|---|---|---|---|---|---|---|---|---|---|---|---|---|---|---|
| Phe | Asn | Leu | Pro | Leu | Gly | Asn | Tyr | Lys | Lys | Pro | Lys | Leu | Leu | Tyr | Cys | Ser | Asn | Gly | Gly |
| TTQ | AAQ | CTN | CCN | CTN | GGN | AAQ | TAQ | AAP | AAP | CCN | AAP | CTN | CTN | TAQ | TGQ | TCN | AAQ | GGN | GGN |
| | | TTP | | TTP | | | | | | | | TTP | TTP | | | AGQ | | | |

| | | | | | 25 | | | | | 30 | | | | | 35 | | | | | 40 |
|---|---|---|---|---|---|---|---|---|---|---|---|---|---|---|---|---|---|---|---|---|
| Tyr | Phe | Leu | Arg | Ile | Leu | Pro | Asp | Gly | Thr | Val | Asp | Gly | Thr | Lys | Asp | Arg | Ser | Asp | Gln |
| TAQ | TTQ | CTN | CGN | ATQ | CTN | CCN | GAQ | GGN | ACN | GTN | GAQ | GGN | ACN | AAP | GAQ | CGN | TCN | GAQ | CAP |
| | | TTP | AGP | ATA | TTP | | | | | | | | | | | AGP | AGQ | | |

| | | | | | 45 | | | | | 50 | | | | | 55 | | | | | 60 |
|---|---|---|---|---|---|---|---|---|---|---|---|---|---|---|---|---|---|---|---|---|
| His | Ile | Gln | Leu | Gln | Leu | Cys | Ala | Glu | Ser | Ile | Gly | Glu | Val | Tyr | Ile | Lys | Ser | Thr | Glu |
| CAQ | ATQ | CAP | CTN | CAP | CTN | TGQ | GCN | GAP | TCN | ATQ | GGN | GAP | GTN | TAQ | ATQ | AAP | TCN | ACN | GAP |
| | ATA | | TTP | | TTP | | | | AGQ | ATA | | | | | ATA | | AGQ | | |

| | | | | | 65 | | | | | 70 | | | | | 75 | | | | | 80 |
|---|---|---|---|---|---|---|---|---|---|---|---|---|---|---|---|---|---|---|---|---|
| Thr | Gly | Gln | Phe | Leu | Ala | Met | Asp | Thr | Asp | Gly | Leu | Leu | Tyr | Gly | Ser | Gln | Thr | Pro | Asn |
| ACN | GGN | CAP | TTQ | CTN | GCN | ATG | GAQ | ACN | GAQ | GGN | CTN | CTN | TAQ | GGN | TCN | CAP | ACN | CCN | AAQ |
| | | | | TTP | | | | | | | | TTP | TTP | | | AGQ | | | |

```
             85                      90                     95                    100
Glu Glu Cys Leu Phe Leu Glu Arg Leu Glu Glu Asn His Tyr Asn Thr Tyr Ile Ser Lys
GAP GAP TGQ CTN TTQ CTN GAP CGN CTN GAP GAP AAQ CAQ TAQ AAQ ACN TAQ ATQ TCN AAP
            TTP     TTP     AGP TTP                                 ATA AGQ

            105                     110                    115                   120
Lys His Ala Glu Lys His Trp Phe Val Gly Leu Lys Lys Asn Gly Arg Ser Lys Leu Gly
AAP CAQ GCN GAP AAP CAQ TGG TTQ GTN GGN CTN AAP AAP AAQ GGN CGN TCN AAP CTN GGN
                                        TTP             AGP AGQ     TTP

            125                     130                    135                   140
Pro Arg Thr His Phe Gly Gln Lys Ala Ile Leu Phe Leu Pro Leu Pro Val Ser Ser Asp
CCN CGN ACN CAQ TTQ GGN CAP AAP GCN ATQ CTN TTQ CTN CCN CTN CCN GTN TCN TCN GAQ
    AGP                             ATA TTP     TTP     TTP         AGQ AGQ
```

Where Q = C or T,

P = A or G, and

N = A, T, C, or G

The nucleotide sequence of the present invention incorporates the following characteristics; codons preferred by Escherichia coli and mammalian cells where possible, elimination of sequences with multiple complementarities, incorporation of unique restriction sites throughout the gene, terminal restriction enzyme sticky ends for ease of inserting the gene into plasmids, a centrally located unique restriction site to allow assembly of the gene in two halves, preferably an N-terminal methionine codon for a translational start site, and tandem translational stop codons.

While the following description and examples illustrate the present invention with respect to a particular nucleotide sequence for bovine aFGF, it is to be understood that the present invention could include any of the permutations listed in Table I. The following table contains the preferred nucleotide sequence:

## TABLE II

TTCAATCTGCCACTGGGTAATTACAAAAAGCCAAAGCTTCTTTACTGCTCTAACGGTGGT 60

TACTTTCTCCGCATCCTGCCAGATGGTACCGTGGACGGCACCAAAGATCGTTCTGATCAA 120

CATATTCAACTGCAGCTGTGCGCCGAATCTATCGGTGAAGTTTACATCAAATCTACCGAA 180

ACTGGTCAATTCCTTGCCATGGACACTGATGGCCTGCTGTACGGATCCCAGACCCCAAAC 240

GAGGAGTGCCTTTTCCTGGAGCGCCTGGAGGAAAACCATTACAACACCTACATCTCTAAA 300

AAGCATGCTGAGAAACATTGGTTCGTAGGCCTTAAGAAAAATGGCCGCTCTAAACTGGGC 360

CCTCGTACTCACTTTGGTCAAAAAGCTATCCTGTTCCTGCCACTGCCAGTGAGCTCTGAC 420

The gene is constructed with a leader portion containing a single restriction enzyme cleavage site and an N-terminal methionine codon for a translational start site. The gene also contains a tail containing tandem translational stop codons and two restriction enzyme cleavage sites. The complementary characteristic of DNA allows a choice of base sequences which in turn allows for the incorporation of unique restriction enzyme cleavage sites throughout the gene. The preferred gene base sequence with the location of the restriction enzyme cleavage sites is shown in the following table:

TABLE III

```
        1                          10                              20                            30
    MetPheAsnLeuProLeuGlyAsnTyrLysLysProLysLeuLeuTyrCysSerAsnGlyGlyTyrPheLeuArgIleLeuProAspGlyThrValAspGlyThrLysAspArgSer


                        [1]                                                              [3]
    1          .          20          .          40          .          60          .          80          .          100          .          120
    AATTCATGTTCAATCTGCCACTGGGTAATTACAAAAAGCCAAAGCTTCTTTACTGCTCTAACGGTGGTTACTTTCTCCGCATCCTGCCAGATGGTACCGTGGACGGCACCAAAGATCGTTCT
    GTACAAGTTAGACGGTGACCCATTAATGTTTTTCGGTTTCGAAGAAATGACGAGATTGCCACCAATGAAAGAGGCGTAGGACGGTCTACCATGGCACCTGCCGTGGTTTCTAGCAAGA
                        [2]                                                              [4]
    (EcoRI)                                          HindIII                                                    KpnI


        40                          50                              60                            70
    AspGlnHisIleGlnLeuGlnLeuCysAlaGluSerIleGlyGluValTyrIleLysSerThrGluThrGlyGlnPheLeuAlaMetAspThrAspGlyLeuLeuTyrGlySerGlnThr


                        [5]                                                              [7]
                .          140          .          160          .          180          .          200          .          220          .          240
    GATCAACATATTCAACTGCAGCTGTGCGCCGAATCTATCGGTGAAGTTTACATCAAATCTACCGAAACTGGTCAATTCCTTGCCATGGACACTGATGGCCTGCTGTACGGATCCAGACC
    CTAGTTGTATAAGTTGACGTCGACACGCGGCTTAGATAGCCACTTCAAATGTAGTTTAGATGGCTTTGACCAGTTAAGGAACGGTACCTGTGACTACCGGACGACATGCCTAGGGTCTGG
                        [6]                                                              [8]
    BclI          PstIPvuII          HinfI                                                    NcoI                          BamHI
```

EP 0 259 953 B1

TABLE III (Cont'd)

```
                                              80                        90                              100                            110
ProAsnGluGluCysLeuPheLeuGluArgLeuGluGluAsnHisTyrAsnThrTyrIleSerLysLysHisAlaGluLysHisTrpPheValGlyLeuLysLysAsnGlyArgSerLys

      [9]      260              280              300              320              340              360
CCAAACGAGGAGTGCCTTTTCCTGGAGCGCCTGGAGGAAAACCATTACAACACCTACATCTCTAAAAAAGCATGCTGAGAAACATTGGTTCGTAGGCCTTAAGAAAAATGGCCGCTCTAAA
GGTTTGCTCCTCACGGAAAAGGACCTCGCGGACCTCCTTTTGGTAATGTTGTGGATGTAGAGATTTTTCGTACGACTCTTTGTAACCAAGCATCCGGAATTCTTTTTACCGGCGAGATTT
                             [10]                       [11]                     [12]
                             HaeII                       SphI                     StuI

                       120                              130                     140
LeuGlyProArgThrHisPheGlyGlnLysAlaIleLeuPheLeuProValSerSerAsp

      [13]        380              400              420              440
CTGGGCCCTCGTACTCACTTTGGTCAAAAGCTATCCTGTTCCTGCCACTGCCAGTGAGCTCTGACTAATAGATATCG
GACCCGGGAGCATGAGTGAAACCAGTTTTCGATAGGACAAGGACGGTGACGGTCACTCGAGACTGATTATCTATAGCAGCT
                     [14]              [15]              [16]
                     ApaI              SacI              EcoRV (SalI)
```

The gene sequence for each strand of the double-stranded molecule is randomly divided into 8 nucleotide sequences. The oligonucleotides are constructed with overlapping ends to allow the formation of the double-stranded DNA. The following table contains one of a multitude of oligonucleotide arrangements that is used to produce the bovine aFGF gene.

9

TABLE IV

```
OLIGO-1     10        20        30        40        50        58
5' AATTCATGTT CAATCTGCCA CTGGGTAATT ACAAAAAGCC AAAGCTTCTT TACTGCTC 3'


OLIGO-2     10        20        30        40      45
5' AGAAGCTTTG GCTTTTTGTA ATTACCCAGT GGCAGATTGA ACATG 3'


OLIGO-3     10        20        30        40        50        60
5' TAACGGTGGT TACTTTCTCC GCATCCTGCC AGATGGTACC GTGGACGGCA CCAAAGATCG 3'


OLIGO-4     10        20        30        40        50        59
5' TGCCGTCCAC GGTACCATCT GGCAGGATGC GGAGAAAGTA ACCACCGTTA GAGCAGTAA 3'


OLIGO-5     10        20        30        40      46
5' TTCTGATCAA CATATTCAAC TGCAGCTGTG CGCCGAATCT ATCGGT 3'


OLIGO-6     10        20        30        40        50        60    65
5' GTAAACTTCA CCGATAGATT CGGCGCACAG CTGCAGTTGA ATATGTTGAT CAGAACGATC TTTGG 3'


OLIGO-7     10        20        30        40        50        60    67
5' GAAGTTTACA TCAAATCTAC CGAAACTGGT CAATTCCTTG CCATGGACAC TGATGGCCTG CTGTACG 3'
```

```
OLIGO-8      10        20        30        40        50        60 62

5' GATCCGTACA GCAGGCCATC AGTGTCCATG GCAAGGAATT GACCAGTTTC GGTAGATTTG AT 3'


OLIGO-9      10        20        30        40        50 52

5' GATCCCAGAC CCCAAACGAG GAGTGCCTTT TCCTGGAGCG CCTGGAGGAA AA 3'


OLIGO-10     10        20        30        40        50        58

5' GTTGTAATGG TTTTCCTCCA GGCGCTCCAG GAAAAGGCAC TCCTCGTTTG GGGTCTGG 3'

OLIGO-11     10        20        30        40        48

5' CCATTACAAC ACCTACATCT CTAAAAAGCA TGCTGAGAAA CATTGGTT 3'


OLIGO-12     10        20        30        40      46

5' GGCCTACGAA CCAATGTTTC TCAGCATGCT TTTTAGAGAT GTAGGT 3'


OLIGO-13     10        20        30        40        50 53

5' CGTAGGCCTT AAGAAAAATG GCCGCTCTAA ACTGGGCCCT CGTACTCACT TTG 3'


OLIGO-14     10        20        39        40        50 55

5' GCTTTTTGAC CAAAGTGAGT ACGAGGGCCC AGTTTAGAGC GGCCATTTTT CTTAA 3'


OLIGO-15     10        20        30        40        50        56

5' GTCAAAAAGC TATCCTGTTC CTGCCACTGC CAGTGAGCTC TGACTAATAG ATATCG 3'


OLIGO-16     10        20        30        40        50

5' TCGACGATAT CTATTAGTCA GAGCTCACTG GCAGTGGCAG GAACAGGATA 3'
```

The oligonucleotides illustrated in Table IV are presented merely as an example of oligonucleotide subunits and should not be construed as limiting thereto. The composite base sequence showing the overlap and arrangement of the oligonucleotides is illustrated in Table III.

The bovine gene is assembled in 2 steps: first, the half corresponding to the N-terminal portion of the protein; and second, the C-terminal half. Generally, the oligonucleotides are kinased with T4 polynucleotide kinase in the presence of either ATP or $^{32}$P-labelled ATP. In the first reaction of each step the oligonucleotides which make up one strand of the gene are kinased with the exception of the most 5' oligonucleotide. In the second reaction the oligonucleotides which make up the second strand are kinased, with the exception of the most 5' oligonucleotide. When kinased oligonucleotides are used, about 1 pmole of the $^{32}$P-labelled oligonucleotide is added for later identification of the products. Annealing is carried out in an appropriate buffer, such as one containing but not limited to about 60 mM TRIS, about pH 7.6, about 5 mM dithiothereitol (DTT), about 10 mM $MgCl_2$, and about 30 $\mu$M ATP at about 90°C for about 4 minutes followed by a rapid transfer to about 60°C and a slow cooling to about 30°C. Ligation is carried out in an appropriate buffer, such as one containing, but not limited to, about 60 mM TRIS, about pH 7.6, about 10 mM DTT, about 10 mM $MgCl_2$, about 1 mM ATP, and about 0.03 units T4 DNA ligase at about 20°C for about 1 and 1/2 hour.

The ligated oligonucleotides are purified by polyacrylamide gel electrophoresis following ethanol precipitation. The oligonucleotides are redissolved in a buffer containing about 20 $\mu$l of about 80%

formamide, about 50 mM TRIS-borate, about pH 8.3, about 1 mM ethylenediaminetetraacetic acid (EDTA), about 0.1% (w/v) xylene cyanol, and about 0.1% (w/v) bromophenol blue. Each sample is heated at about 90°C for about 3 minutes and electrophoresed in about a 10% urea-polyacrylamide gel at about 75 watts for about 5 hours. The 231 base N-terminal bands are removed, combined and eluted at about 4°C in about 0.5 M ammonium acetate containing about 1mM EDTA at about pH 8. The 209 base C-terminal bands are treated in the same manner.

The synthetic gene sequences coding for either the N-terminal or the C-terminal portions of the aFGF are incorporated into the pBR322 plasmid. It is especially desired and intended that there be included within the scope of this invention, the use of other plasmids into which the aFGF gene can be incorporated and which will allow the expression of the aFGF gene. Reannealed oligonucleotides, about 300 fmole and about 100 fmole of the recovered 231 base pair N-terminus are each ligated to about 100 fmole of agarose gel purified about 3.9 kilo base (kb) EcoRI-BamHI pBR322 for the N-terminus. The 209 bp C-terminus is constructed in the same manner using BamHI-SalI pBR322. Ligation is carried out in a buffer containing about 25 mM TRIS, about pH 7.8, about 1 mM DTT, about 10 mM MgCl$_2$, about 0.4 mM ATP, with about 1 unit of T4 DNA ligase for about 1 hour at about 20°C. Each half-gene ligated vector is used to transform competent bacterial cells, such as E. coli RRI (Bethesda Research Laboratories, BRL) following suppliers procedures. The transformed cells are selected for growth in ampicillin and screened for the presence of either the 231 base pair (bp) EcoRI-BamHI insert or the 209 bp BamHI-SalI insert by restriction analysis of mini-lysate plasmid preparations.

The DNA sequence of clones containing the appropriate sized inserts is determined using Maxam and Gilbert, Proc. Natl. Acad. Sci. USA 74: 560-564 (1977) chemical DNA sequence techniques.

The final full-length aFGF synthetic gene was cloned by cleaving the N-terminal half clone with restriction enzymes BamHI and SalI, treating with alkaline phosphatase and ligating this to the gel purified 209 bp BamHI-SalI insert of the C-terminal half clone. This ligated material was used to transform competent RRI cells as before.

Expression of the synthetic aFGF gene is accomplished by a number of different promoter-expression systems. It is desired and intended that there be included within the scope of this invention, the use of other promoter-expression systems for the expression of the intact aFGF gene. The preferred construct uses the E. coli tac promoter, a hybrid between regions of the trp promoter and the lac promoter as described by deBoer et al., Proc. Nat. Acad. Sci. USA 80: 21-25 (1983). Plasmid pKK 223-3 (Pharmacia) which contains the tac promoter and rrnB rRNA transcription terminator was modified to remove the pBR322-derived SalI restriction enzyme site. The rrnB rRNA terminator has been shown to allow expression by strong promoters, Gentz et al., Proc. Natl. Acad. Sci. USA 78: 4936-4940 (1981); Brosius, Gene 27: 161-172 (1984).

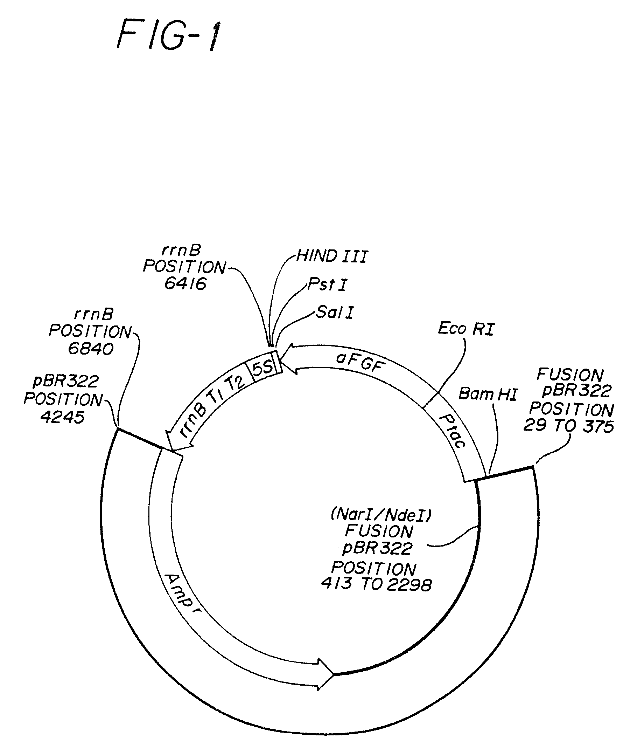

The pKK223-3 plasmid DNA is cleaved with restriction enzymes to produce a 2.7 kb DNA fragment to generate clone pKK 2.7. The synthetic aFGF gene is cleaved from its pBR322 vector and transferred to the pKK 2.7 plasmid after restricting pKK 2.7 with EcoRI and SalI. The resulting recombinant, shown in figure 1, is transformed into E. coli JM105 (Pharmacia) or DH5 (BRL) cells and expressed.

Site specific mutagenesis is an efficient way to convert the amino acid sequence of one mammalian species of aFGF to the aFGF amino acid sequence of another species. The following description relates to the site specific mutagenic conversion of bovine aFGF, 140 amino acid form, to human aFGF, it is to be understood, however, that the process can be used to convert any mammalian species aFGF to that of any other species. The only limitation on the conversion is that the amino acid sequences of both aFGFs must be known. The following table lists the amino acids which must be substituted and the location on the bovine aFGF amino acid map, Table III, at which the substitutions are made:

TABLE V

| Amino Acid Location | Substituted Amino Acids | |
|---|---|---|
| | Human aFGF | for Bovine aFGF |
| 5 | Pro | Leu |
| 21 | His | Tyr |
| 35 | Arg | Lys |
| 47 | Ser | Cys |
| 51 | Val | Ile |
| 64 | Tyr | Phe |
| 106 | Asn | His |
| 116 | Ser | Arg |
| 117 | Cys | Ser |
| 119 | Arg | Leu |
| 125 | Tyr | Phe |

As with the bovine gene sequence eight oligonucleotides representing the human gene sequence are constructed by the same procedure as that used for the bovine oligonucleotides. The following table contains one of a multitude of oligonucleotide arrangements that is used to produce the human aFGF gene.

TABLE VI

OLIGO-1
5' CTGCCACCGGGTAATTAC 3'
OLIGO-2
5' CGGTGGTCACTTTCTCCG 3'
OLIGO-3
5' CGGCACCAGAGATCGTTC 3'
OLIGO-4
5' GCAGCTGTCCGCCGAATCTGTCGGTGAAG 3'
OLIGO-5
5' CTGGTCAATACCTTGCCATGG 3'
OLIGO-6
5' GCTGAGAAAAATTGGTTCG 3'
OLIGO-7
5' GGCCGCGTTTACAGCTGCCATTTTTCTTAAGG 3'
OLIGO-8
5' CGTACTCACTATGGCCAAAAAGCTATCC 3'

The cloned synthetic bovine gene for aFGF is converted to a human synthetic gene for aFGF by a series of directed point mutations. Oligonucleotide-directed mutagenesis of the cloned gene allows the alteration of the base sequence of bovine aFGF so that the resulting amino acid sequence contains the substituted amino acids shown in Table V and is human aFGF. A deletion is made in the bovine, gene to remove the amino terminal phenylalanine for the production of the human 139 amino acid micro-heterogeneous form of aFGF. A point mutation is carried out to replace the second position asparagine with aspartic acid. Alternatively, the asparagine is deamidated to aspartic acid. The methods for carrying out these procedures are described below or are known in the art. The oligonucleotide-directed mutagenesis is carried out using standard procedures known to the art, Zoller and Smith, Methods in Enzymology, 100: 468-500 (1983); Norris et al., Nucleic Acids Research, 11: 5103-5112 (1983); and Zoller and Smith, DNA, 3: 479-488 (1984). The point mutations carried out by the standardized oligonucleotide-directed mutagenesis are shown in the following, Table VII. The location of the base mutagenesis can be seen in Table III. The point mutations are presented merely as an example of changes which will result in the human aFGF gene and should not be construed as limiting thereto.

TABLE VII

| Base Location | Substituted Base | | Corresponding Human Amino Acid |
|---|---|---|---|
| | Human aFGF | for Bovine aFGF | |
| 22 | C | T | Pro |
| 69 | C | T | His |
| 112 | G | A | Arg |
| 148 | C | G | Ser |
| 159 | G | A | Val |
| 199 | A | T | Tyr |
| 324 | A | C | Asn |
| 354 | A | C | Ser |
| 358 | G | C | Cys |
| 364 | G | T | Arg |
| 365 | C | G | Arg |
| 382 | A | T | Tyr |

The expression clones are grown at about 37°C in an appropriate growth medium, which consists of about 1% tryptone, about 0.5% yeast extract, about 0.5% NaCl, about 0.4% glucose and about 50 $\mu$g/ml ampicillin. When the optical density at 550 nm reaches about 0.5, isopropyl-$\beta$-D-thiogalactopyranoside (IPTG) may be added to give a final concentration of about 1 mM and growth is continued at about 37°C for about 3 hours. The cells from 1 liter of culture medium are harvested by centrifugation and resuspended in a disruption buffer containing about 10 mM sodium phosphate at about pH 7.2, about 5 mM EDTA, about 10.6 $\mu$g/ml N-p-toluenesulfonyl-L-phenylalanine chloromethyl ketone (TPCK), about 34.3 $\mu$g/ml pepstatin A, about 87 $\mu$g/ml phenylmethylsulfonyl fluoride (PMSF), about 15 $\mu$g/ml bovine pancreatic trypsin inhibitor (BPTI), and about 25.2 $\mu$g/ml leupeptin. The cells are either immediately disrupted or frozen and stored at -70°C and disrupted immediately after thawing by about three passages through a French pressure cell at about 12,000 psi at about 4°C. The supernatant fluid is collected by centrifugation.

The recombinant aFGF is purified to homogeneity by a unique two-step chromatographic procedure employing a combination of heparin-Sepharose affinity chromatography followed by reversed-phase high performance liquid chromatography (HPLC). The crude r-aFGF is loaded onto a heparin-Sepharose column in a dilute buffer such as about 10 mM phosphate or Tris, about pH 6 to 8, which is subsequently washed with a low concentration of salt, such as about 0.8 M NaCl, until the absorbance at 280 nm drops to about background. The r-aFGF is eluted with a buffered high salt concentration solution such as about 10 mM sodium phosphate or Tris, about pH 6 to 8, containing about 1.5 M NaCl. The eluate is then purified by reversed-phase HPLC on a resin consisting of covalently linked alkyl silane chains with alkyl groups having from 3 to 18 carbon atoms, preferably 4 carbon atoms. The r-aFGF is directly applied to the HPLC column equilibrated in a dilute acid such as about 10 mM trifluoroacetic acid, acetic acid or phosphoric acid and eluted with a linear gradient of organic solvent such as acetonitrile or ethanol. Bovine brain-derived aFGF was previously described to bind to both heparin-Sepharose by Maciag et al. Science 225: 932-935 (1984) and to reversed-phase HPLC columns by Thomas et al. Proc. Natl. Acad. Sci. USA 81: 357-361 (1984) as part of multistep purification protocols. Based, in part, on the relatively high abundance of r-aFGF in bacterial lysates, these two steps alone are herein demonstrated to be sufficient to obtain homogeneously pure r-aFGF of about 16,000 daltons as established by electrophoresis in polyacrylamide gels. These two steps alone do not yield pure aFGF from brain.

Mitogenic activity of the purified aFGF is determined by the incorporation of $^3$H-thymidine into DNA by cell line fibroblasts, preferably BALB/c 3T3 A31 (American Type Culture Collection). The recombinant aFGF shows a peak response at about 1 ng protein or less per ml in the fibroblast stimulative assay.

Another embodiment of this invention is a method of promoting the healing of wounds by application of the novel peptide, either with or without heparin, preferably with heparin, about 1 to about 500 $\mu$g/cm$^2$ of this invention to the wound area either topically or subcutaneously in the wound in an amount of about 0.1 to 100 $\mu$g/cm$^2$ of surface for topical application.

For application, various pharmaceutical formulations are useful such as ointments, pastes, solutions, gels, solid water soluble polymers such as albumins, gelatins, hydroxypropyl cellulose, pluronics, tetronics or alginates in which the active ingredient is incorporated in amounts of about 1 to about 100 $\mu$g/ml.

The ability of aFGF to stimulate division in various cell types including fibroblasts, vascular and corneal endothelial cells and the like makes these peptides useful as pharmaceutical agents. These compounds can be used to treat wounds of mammals including humans by the administration of the novel r-aFGF to patients in need of such treatment.

The following examples illustrate the present invention without, however, limiting the same thereto.

EXAMPLE 1

Oligonucleotide Synthesis

Oligonucleotides were synthesized according to the technique described by Matteucci and Caruthers, J. Am. Chem. Soc. 103: 3185-3191 (1981); Beaucage and Caruthers, Tetrahedron Letters 22: 1859-1862 (1981). The base sequences of the synthesized oligonucleotides are shown in Table IV.

EXAMPLE 2

Assembly of the aFGF Gene

The oligonucleotides from Example 1 were assembled as two separate units, the N-terminal half (231 bp) and the C-terminal half (209 bp). The two halves were then combined for the intact synthetic gene, see Table III. Initially the oligonucleotides were kinased in the following reaction mixture: 70 mM Tris pH 7.6, 5 mM DTT, 10 mM $MgCl_2$, 33 $\mu M$ ATP, 0.3 units T4 polynucleotide kinase per $\mu l$, and 2.5 pmole oligonucleotide per $\mu l$. The mixture was incubated 1.5 hours at 37°C and then an additional hour after supplementing the mixture with 0.2 units/$\mu l$ kinase and ATP to give a concentration of 100 mM. For radioactive labelling, the initial mixture contained 37 nCi/$\mu l$ of [$\gamma$-$^{32}$P]-ATP.

The annealing and ligations were done in two separate reactions. In each reaction, 100 pmole of each of the eight oligonucleotides were added. In one reaction the oligonucleotides which make up one strand of the C-terminal or N-terminal half gene were kinased with the exception of the most 5' oligonucleotide. In the second reaction the oligonucleotides which make up the opposite strand were kinased, again with the exception of the most 5' oligonucleotide. Thus, in each reaction 3 oligonucleotides were kinased and 5 were not. When kinased oligonucleotides were used, 1 pmole of the $^{32}$P-labelled oligonucleotide was also added for later identification of the products. Each reaction contained 200 $\mu l$ with 70 mM Tris pH 7.6, 5 mM DTT, 10 mM $MgCl_2$, and 30 $\mu M$ ATP. The oligonucleotides were annealed by heating to 90°C for 4 minutes, then immediately transferring the reaction to 60°C and allowing it to cool slowly to 30°C. Ligation was done in 400 $\mu l$ containing 60 mM Tris pH 7.6, 10 mM DTT, 10 mM $MgCl_2$, 1 mM ATP, and 0.03 units T4 DNA ligase per $\mu l$ by incubating at 20°C for 1.5 hours.

Polyacrylamide gel electrophoresis was used to purify the ligated oligonucleotides. The ligated oligonucleotides were precipitated with ethanol, redissolved in 20 $\mu l$ of 80% formamide, 50 mM TRIS-borate pH 8.3, 1 mM EDTA, 0.1% (w/v) xylene cyanol, and 0.1% (w/v) bromophenol blue. Each sample was heated at 90°C for 3 minutes and electrophoresed in a 10% urea-polyacrylamide gel at 75 watts for 5 hours. The oligonucleotide bands were visualized by exposing the gel to X-ray film.

The 231 base bands of each reaction for the N-terminus were cut out of the gel, combined, and eluted at 4°C in 1 ml of 0.5 M ammonium acetate, 1 mM EDTA pH 8. The eluted DNA was precipitated with ethanol and redissolved in 30 $\mu l$ of 70 mM Tris pH 7.6, 5 mM DTT, and 10 mM $MgCl_2$. The 209 base bands of the C-terminus were eluted in the same manner.

The gel purified oligonucleotides were annealed prior to transformation by heating to 90°C for 4 minutes and slow cooling to 20°C. Assuming a 5% recovery from the initial starting oligonucleotides, 300 fmole and 100 fmole of recovered annealed 231 bp oligonucleotides were each ligated to 100 fmole of agarose gel purified 3.9 kb EcoRI-BamHI pBR322 fragment DNA in 20 $\mu l$ of 25 mM Tris pH 7.8, 1 mM DTT, 10 mM $MgCl_2$, 0.4 mM ATP, with 1 unit T4 DNA ligase for 1 hour at 20°C. The annealed 209 bp oligonucleotides were ligated to agarose purified 3.9 kb BamHI-SalI pBR322 fragment DNA under the same conditions as the 231 base pair fragments. The ligation reactions were diluted 1:5 in $H_2O$ and 1 $\mu l$ of dilution was used to transform 20 $\mu l$ of competent E. coli RRI cells (BRL) as described by the supplier. The transformants were selected for growth in ampicillin and screened for the presence of the 231 bp EcoRI-BamHI or the 209 bp BamHI-SalI insert by restriction analysis of mini-lysate plasmid preparations.

The DNA sequence of clones containing the appropriate sized inserts was determined using the chemical DNA sequence techniques of Maxam and Gilbert, Proc. Natl. Acad. Sci. USA 74: 560-564 (1977). Since none of the 231 bp clones had the correct sequence, a clone containing the correct sequence was prepared as follows. One clone with the correct sequence between the KpnI and BamHI sites was cleaved with KpnI and with SalI, which cleaves in the pBR322 vector. The 400 bp band was gel purified and ligated to the 3.8 kb KpnI-SalI band of a second clone containing the correct sequence from the EcoRI site to the KpnI site of the aFGF gene insert. After transformation, a resulting clone was sequenced to ensure the desired sequence had been obtained.

Since a clone containing the correct 209 bp sequence was obtained, no further manipulation of these clones was required. The final full-length aFGF synthetic gene was cloned by cleaving the N-terminal half clone with BamHI and SalI, treating with alkaline phosphatase, and ligating this to the gel purified 209 bp BamHI-SalI insert of the C-terminal half clone. This ligated material was used to transform competent RRI cells as before.

EXAMPLE 3

Expression of the Synthetic Bovine aFGF Gene

The intact aFGF gene from Example 2 was incorporated into a modified pKK223-3 plasmid. The pKK223-3 plasmid (Pharmacia) contains the tac promoter which is a hybrid between regions of the trp promoter and the lac promoter, deBoer et al., Proc. Natl Acad. Sci. USA 80: 21-25 (1983). This plasmid also contains the rrnB rRNA transcription terminator, a strong terminator sequence found to allow expression from strong promoters, Gentz et al., Proc. Natl. Acad. Sci. USA 78: 4936-4940 (1981); Brosius, Gene 27: 161-172 (1984). The pKK 223-3 plasmid was modified to remove the pBR322-derived SalI restriction enzyme site. This was accomplished by cleaving the pKK223-3 plasmid DNA with NdeI and NarI, and recircularizing the 2.7 kb DNA fragment to generate clone pKK2.7. The synthetic aFGF gene was then cleaved from its pBR322 vector and transferred to pKK2.7 after restricting this expression vector with EcoRI and SalI. This construction positions the initiating methionine of the synthetic gene 11 bases downstream of the Shine-Dalgarno ribosome binding site. The resulting recombinant, shown in Figure 1, was transformed into E. coli JM105 cells and also into E. coli DH5 cells.

The expression clones were grown at 37°C in LB broth (1% tryptone, 0.5% yeast extract, 0.5% NaCl) containing 0.4% glucose and 50 $\mu$g/ml ampicillin. When the optical density at 550 nm reached 0.5, IPTG was added to give 1 mM and growth was continued at 37°C for 3 hours. The cells were harvested by centrifugation at 10,000 x g for 20 minutes and the cells from 1 liter of culture were resuspended in 20 ml of 10mM sodium phosphate pH 7.2, (heparin-Sepharose buffer) 5 mM EDTA, 10N6 $\mu$g/ml TPCK, 34.3 $\mu$g/ml pepstatin A, 87 $\mu$g/ml PMSF, 15 $\mu$g/ml BPTI, and 34.3 $\mu$g/ml leupeptin. The resuspended cells were quickly frozen in a dry ice/ethanol bath and stored overnight at -70°C.

EXAMPLE 4

Extraction and Purification of Recombinant aFGF

The frozen cells from Example 3 were thawed, an additional 87 $\mu$g/ml PMSF was added, and the preparation was passed through a French pressure cell at 12,000 psi three times at 4°C. The resulting lysate was centrifuged at 93,000 x g for 30 minutes to remove cell debris. The supernatant was removed, adjusted to pH 7.2 with 1 M NaOH and loaded onto a 1.6 x 10 cm heparin-Sepharose (Pharmacia) column run at 4°C with a flow rate of 20 ml per hour collecting 2 ml fractions. The pellet was resuspended in 5 ml of 10 mM sodium phosphate, 2 M NaCl, pH 7.2, recentrifuged at 93,000 x g for 30 minutes and the supernatant diluted with three volumes of 10 mM sodium phosphate, pH 7.2, readjusted to pH 7.2 with 1 M NaOH, if necessary, and loaded onto the same heparin-Sepharose column. After loading, the column was washed with 10 mM sodium phosphate, 0.8 M NaCl, pH 7.2 until the absorbance at 280 nm fell to background. Bound r-aFGF was eluted as a single peak with 10 mM sodium phosphate, 1.5 M NaCl, pH 7.2. The pooled fractions from the heparin-Sepharose column were purified by reversed-phase HPLC using a 4.6 mm x 25 cm $C_4$ column (Separations Group) as described by Thomas et al., Proc. Natl. Acad. Sci. USA 81: 357-361 (1984). The r-aFGF eluted as a single major peak that was resolved from multiple minor contaminant peaks suggesting that the protein was homogeneously pure. Polyacrylamide gel electrophoresis was used to confirm purity. The purified r-aFGF was electrophoresed following the technique of O'Farrell, J. Biol. Chem. 250: 4007-4021 (1975). Silver staining revealed a single band with a molecular mass of 16,000 daltons. Identity of the protein as aFGF was confirmed by both amino acid analysis and amino terminal sequence determination.

EXAMPLE 5

Biological Activity of Bovine Recombinant aFGF

Biological activity of the purified r-aFGF from Example 4 was evaluated using a fibroblast mitogenic assay as described by Thomas et al., J. Biol. Chem. 225: 5517-5520 (1980). BALB/c 3T3 A31 fibroblasts (American Type Culture Collection) were plated at 2 x 10⁴ cells per 35 mm diameter well in culture media containing 10% heat-inactivated calf serum and incubated in 7% $CO_2$ (pH 7.35 ± 0.05). The cells became fully quiescent by replacing the media with 0.5% heat-inactivated calf serum 6 and again 24 hours later. At 55 hours after plating, 50 $\mu$g of heparin, test samples and 1.1 $\mu$g of dexamethasone were added, at 70 hours each well was supplemented with 2 $\mu$Ci of [methyl-³H]-thymidine (20 Ci/mmole, New England Nuclear) and 3 $\mu$g of unlabeled thymidine (Sigma), and at 95 hours the cells were processed for determination of radiolabel incorporated into DNA. Each dose-response point was the average of triplicate determinations. The results are shown in the following table:

TABLE VIII

| Mitogenic Responses of BALB/c 3T3 Fibroblasts to Bovine r-aFGF | | |
|---|---|---|
| Concentration r-aFGF (ng/ml) | CPM | |
| | r-aFGF | Brain aFGF |
| 0.003 | 268 | 231 |
| 0.010 | 498 | 329 |
| 0.031 | 1550 | 1017 |
| 0.100 | 7031 | 3684 |
| 0.316 | 9319 | 11353 |
| 1.000 | 4718 | 9050 |

The activity of the recombinant aFGF was equal to or slightly greater than that of brain derived aFGF. The purified r-aFGF had a half-maximal stimulation of DNA synthesis at about 71 pg/ml while purified brain derived aFGF had a half-maximal value 126 pg/ml.

EXAMPLE 6

Mutagenesis of the Bovine aFGF Gene to the Human aFGF Gene

To facilitate the mutagenesis of the bovine aFGF gene, the synthetic gene from Example 2 was transferred to M13mp19, a single-stranded DNA bacteriophage vector. Standard mutagenesis procedures were used as reported by Zoller and Smith, Methods in Enzymology, 100: 468-500 (1983); Norris et al., Nucleic Acids Research, 11: 5103-5112; and Zoller and Smith, DNA, 3: 479-488. The bovine pKK-aFGF plasmid was cleaved with EcoRI and SalI, see Table III, and the resulting 440 bp fragment was agarose gel purified as in Example 2. Vector M13mp19 RF DNA (BRL) was cleaved with the same two endonucleases and the ends were subsequently dephosphorylated in 100 $\mu$l of 10 mM Tris pH 8.0 buffer with 100 units of bacterial alkaline phosphatase. A ligation was performed using 50 ng of the treated vector DNA and 12 ng of the aFGF gene fragment DNA in 10 $\mu$l of 25 mM Tris pH 7.8, 10 mM $MgCl_2$, 1 mM DTT, 0.4 mM ATP, with 2 units of T4 DNA ligase for 16 hours at 4°C. The reaction mixture was diluted 1:5 in $H_2O$ and 1 $\mu$l of dilution was used to transform 20 $\mu$l of competent E. coli DH5 cells (BRL) as described by the supplier. The cells were plated with E. coli JM105 (Pharmacia) host cells in 0.03% X-gal and 0.3 mM IPTG; after incubation at 37°C colorless plaques were isolated. One phage clone containing the bovine aFGF gene was selected, M13mp19-aFGF.

Eight oligonucleotides were designed to specify the human sequence and synthesized, see Table VI.

Oligomer 8 contains an additional mutation in which thymine at site 386 in the bovine gene is replaced by cytosine in the human gene. This mutation allows the incorporation of a restriction site without altering the human aFGF amino acid sequence.

The human oligomers 1, 2, 3, 4, 6, and 8 were phosphorylated and 15 pmoles of each were annealed individually to 0.5 pmole of M13mp19-aFGF single-stranded phage DNA in 10 $\mu$l of 20 mM Tris pH 7.5, 10 mM $MgCl_2$, 50 mM NaCl, 1 mM DTT for 10 minutes at 65°C followed by 10 minutes at 23°C. Closed-circular double-stranded molecules were then prepared in 20 $\mu$l of 20 mM Tris pH 7.5, 10 mM MgCl$_2$, 25 mM NaCl, 5.5 mM DTT, 0.5 mM ATP, 0.25 mM dATP, 0.25 mM dCTP, 0.25 mM dCTP, 0.25 mM dGTP, 0.25 mM dTTP, using 1 unit of T4 DNA ligase and 2 units of DNA polymerase I klenow fragment by incubation at 15°C for 17 hours. The preparations were each used to transform competent JM105 cells and the resulting transformant plaques were selected by hybridization with the appropriate oligomer which had been radio-labeled using $^{32}$P-ATP and polynucleotide kinase. The conditions of hybridization were optimized for each probe to prevent formation of hybrids containing single base changes. Single-stranded DNA was isolated from the phage clone containing the human oligomer 4 mutations and the above procedure was repeated using the human oligomer 5 to generate a clone containing both the oligomer 4 and 5 mutations.

In the following procedures the bovine-to-human sequence mutations in these M13-based clones were combined into one pBR322-based clone. RF DNAs were prepared from clones containing the base changes specified by human oligomers 1, 2, 6, and 8. The DNA of the human 1 mutant clone was cleaved with EcoRI, the ends were dephosphorylated with bacterial alkaline phosphatase, and the DNA was cleaved with HindIII. The human 2 mutant DNA was cleaved with HindIII, treated with phosphatase, and then cleaved with BamHI. The human 6 mutant DNA was cleaved with BamHI, phosphatase treated, and subsequently cleaved with ApaI. Likewise, the human 8 mutant DNA was cleaved with ApaI, the ends were dephosphorylated, and the DNA was cleaved with SalI. These four DNA preparations were electrophoresed through 2% agarose and the fragments of 45 bp, 190 bp, 135 bp, and 70 bp from the mutant DNAs containing human 1, 2, 6, and 8 mutations, respectively, were eluted from the gel. Approximately 60 fmoles of each fragment were collectively ligated to about 60 fmoles of a gel-purified 3.7 kb EcoRI-SalI fragment from pBR322 in 5 $\mu$l of 25 mM Tris pH 7.8, 10 mM MgCl$_2$, 1 mM DTT, 0.4 mM ATP, with 1.5 units of T4 DNA ligase for 16 hours at 12°C The reaction mixture was diluted 1:5 in H$_2$0 and 1 $\mu$l of dilution was used to transform 20 $\mu$l of competent E. coli DH5 cells (BRL) as described by the supplier. A clone containing the mutations specified by all four mutant oligomers was selected by hybridization with radiolabeled probes prepared from each of the oligomers. The 140 bp KpnI-BamHI DNA fragment isolated from cleaved RF DNA of the human 3 mutant M13 clone was ligated to endonuclease cleavage products of this human 1-2-6-8 mutant DNA and transformed into DH5 competent cells to generate a clone with the human 1-2-3-6-8 mutations. BamHI-PstI digestion fragments of this latter clone were ligated to the BamHI-PstI digestion fragments of RF DNA from the human 4-5 M13-based clone and the ligation mixture was used to transform DH5 competent cells. A clone containing the human 1-2-3-4-5-6-8 mutations was selected by oligomer hybridization and the aFGF gene EcoRI-SalI DNA fragment of this recombinant plasmid was ligated to phosphatase-treated EcoRI-SalI-cleaved RF DNA of M13mp18 (BRL). Competent DH5 cells were transformed with this ligated DNA and the transformed cells were plated on JM105 host cells to generate an M13 clone. The single-stranded phage DNA of this clone was annealed with the human 7 oligomer and an M13 clone containing all the desired mutations was obtained following the procedure described above. RF DNA was prepared from this clone and cleaved with EcoRI and SalI. The resulting 440 bp band was gel purified and ligated to the 2.7 kb EcoRI-SalI DNA fragment of the pKK2.7 tac promoter expression vector. This DNA was used to transform competent DH5 cells thus generating the human pKK-aFGF expression clone used for production of the human form of aFGF.

The human r-aFGF was purified by the same procedure as that used for the bovine r-aFGF, see Example 4. The human r-aFGF was judged to be at least 99.75% pure based on the presence of a single intense band on a silver stained SDS electrophoretic gel loaded with 400 ng of purified human r-aFGF and having a sensitivity of about 1 ng/band. The protocol is described in Example 4.

The pure recombinant human aFGF was assayed for mitogenic activity using $^3$H-thymidine incor-poration into subconfluent BALB/c 3T3 cells as described for the bovine recombinant protein in Example 5. As previously observed with human brain-derived aFGF assayed on vascular endothelial cells, the recombinant human protein shows a greater difference in the heparin (50 $\mu$g/ml) activation than does either the brain-derived or recombinant bovine aFGF, Gimenez-Gallego et al. Biochem. Biophys. Res. Comm. 135: 541-548(1986); the results of recombinant human aFGF on Balb/c 3T3 cells are shown in the following table:

TABLE IX

| Mitogenic Responses of BALB/c 3T3 Fibroblasts to Human r-aFGF. | | |
|---|---|---|
| Concentration r-aFGF (picograms/ml)* | CPM | |
| | - heparin | + heparin |
| 0 | 3574 | 991 |
| 1 | 4156 | 1336 |
| 3.16 | 4216 | 1802 |
| 10.0 | 4092 | 2617 |
| 31.6 | 4155 | 4824 |
| 100 | 4274 | 10489 |
| 316 | 6060 | 14584 |
| 1000 (1 ng) | 6811 | 10547 |
| 3160 | 7910 | 12357 |
| 10000 | 8597 | 9143 |
| 31600 | 9700 | 9057 |
| 100000 | 11166 | 9277 |
| 1000000 (1 $\mu$g) | 15864 | 12425 |

*picogram $= 10^{-12}$ grams

In the presence of heparin, the half-maximal stimulation occurs at about 42 pg/ml. In the absence of heparin the peak has not clearly been reached even at the highest concentration but must be greater than about 30 ng/ml.

**Claims**
**Claims for the following Contracting States : AT, BE, CH, DE, FR, GB, IT, LI, LU, NL, SE**

1. A nucleotide sequence coding for the human recombinant acidic fibroblast growth factor having an amino acid sequence of:

PheAsnLeuProProGlyAsnTyrLysLysProLysLeuLeuTyrCysSerAsnGlyGlyHisPheLeuArgIleLeu

ProAspGlyThrValAspGlyThrArgAspArgSerAspGlnHisIleGlnLeuGlnLeuSerAlaGluSerValGlyGlu

ValTyrIleLysSerThrGluThrGlyGlnTyrLeuAlaMetAspThrAspGlyLeuLeuTyrGlySerGlnThrProAsn

GluGluCysLeuPheLeuGluArgLeuGluGluAsnHisTyrAsnThrTyrIleSerLysLysHisAlaGluLysAsnTrp

PheValGlyLeuLysLysAsnGlySerCysLysArgGlyProArgThrHisTyrGlyGlnLysAlaIleLeuPheLeuPro

LeuProValSerSerAsp .

wherein there is attached to the phenylaianine at the first position a methionine, wherein the base sequence is:

EP 0 259 953 B1

```
        1              20                40                60                80
AATTCATGTTCAATCTGCCACCGGGTAATTACAAAAAGCCAAAGCTTCTTTACTGCTCTAACGGTGGTCACTTTCTCCGC
   GTACAAGTTAGACGGTGGCCCATTAATGTTTTTCGGTTTCGAAGAAATGACGAGATTGCCACCAGTGAAAGAGGCG


              100              120              140               160
 ATCCTGCCAGATGGTACCGTGGACGGCACCAGAGATCGTTCTGATCAACATATTCAACTGCAGCTGTCCGCCGAATCTGT
   TAGGACGGTCTACCATGGCACCTGCCGTGGTCTCTAGCAAGACTAGTTGTATAAGTTGACGTCGACAGGCGGCTTAGACA


             180              200             ·220               240
CGGTGAAGTTTACATCAAATCTACCGAAACTGGTCAATACCTTGCCATGGACACTGATGGCCTGCTGTACGGATCCCAGA
   GCCACTTCAAATGTAGTTTAGATGGCTTTGACCAGTTATGGAACGGTACCTGTGACTACCGGACGACATGCCTAGGGTCT


             260              280              300               320
 CCCCAAACGAGGAGTGCCTTTTCCTGGAGCGCCTGGAGGAAAAACCATTACAACACCTACATCTCTAAAAAGCATGCTGAG
   GGGGTTTGCTCCTCACGGAAAAGGACCTCGCGGACCTCCTTTTGGTAATGTTGTGGATGTAGAGATTTTTCGTACGACTC


             340              360              380               400
 AAAAATTGGTTCGTAGGCCTTAAGAAAAATGGCAGCTGTAAACGCGGCCCTCGTACTCACTATGGCCAAAAAGCTATCCT
   TTTTTAACCAAGCATCCGGAATTCTTTTTACCGTCGACATTTGCGCCGGGAGCATGAGTGATACCGGTTTTTCGATAGGA


             420              440
GTTCCTGCCACTGCCAGTGAGCTCTGACTAATAGATATCG
   CAAGGACGGTGACGGTCACTCGAGACTGATTATCTATAGCAGCT.
```

2. An expression plasmid comprising the nucleotide sequence of Claim **1** inserted therein.

3. A host that is compatible with and contains the plasmid of Claim **2**.

4. The plasmid of Claim **2** which is capable of expressing the gene for human acidic fibroblast growth factor.

5. A method of purifying recombinant acidic fibroblast growth factor (aFGF) in pure form comprising the following steps:
   a. partial purification of recombinant aFGF by a heparin-Sepharose affinity chromatography matrix and an acceptable eluant; followed by
   b. final purification of partially purified recombinant aFGF by reverse phase high performance liquid chromatography using an alkyl silane substrate and an acceptable eluant.

**Claims for the following Contracting States : ES, GR**

1. A process for the preparation of a nucleotide sequence coding for the human recombinant acidic fibroblast growth factor having an amino acid sequence of:

21

PheAsnLeuProProGlyAsnTyrLysLysProLysLeuLeuTyrCysSerAsnGlyGlyHisPheLeuArgIleLeu

ProAspGlyThrValAspGlyThrArgAspArgSerAspGlnHisIleGlnLeuGlnLeuSerAlaGluSerValGlyGlu

ValTyrIleLysSerThrGluThrGlyGlnTyrLeuAlaMetAspThrAspGlyLeuLeuTyrGlySerGlnThrProAsn

GluGluCysLeuPheLeuGluArgLeuGluGluAsnHisTyrAsnThrTyrIleSerLysLysHisAlaGluLysAsnTrp

PheValGlyLeuLysLysAsnGlySerCysLysArgGlyProArgThrHisTyrGlyGlnLysAlaIleLeuPheLeuPro

LeuProValSerSerAsp .

wherein there is attached to the phenylalanine at the first position a methionine, wherein the base sequence is:

```
        1              20            40            60            80
AATTCATGTTCAATCTGCCACCGGGTAATTACAAAAAGCCAAAGCTTCTTTACTGCTCTAACGGTGGTCACTTTCTCCGC
       GTACAAGTTAGACGGTGGCCCATTAATGTTTTTTCGGTTTCGAAGAAATGACGAGATTGCCACCAGTGAAAGAGGCG


                100           120           140           160
 ATCCTGCCAGATGGTACCGTGGACGGCACCAGAGATCGTTCTGATCAACATATTCAACTGCAGCTGTCCGCCGAATCTGT-
      TAGGACGGTCTACCATGGCACCTGCCGTGGTCTCTAGCAAGACTAGTTGTATAAGTTGACGTCGACAGGCGGCTTAGACA


                180           200          ·220          240
CGGTGAAGTTTACATCAAATCTACCGAAACTGGTCAATACCTTGCCATGGACACTGATGGCCTGCTGTACGGATCCCAGA
     GCCACTTCAAATGTAGTTTAGATGGCTTTGACCAGTTATGGAACGGTACCTGTGACTACCGGACGACATGCCTAGGGTCT.


                260           280           300           320
CCCCAAACGAGGAGTGCCTTTTCCTGGAGCGCCTGGAGGAAAACCATTACAACACCTACATCTCTAAAAAGCATGCTGAG
    GGGGTTTGCTCCTCACGGAAAAGGACCTCGCGGACCTCCTTTTGGTAATGTTGTGGATGTAGAGATTTTTCGTACGACTC


                340           360           380           400
AAAAATTGGTTCGTAGGCCTTAAGAAAAATGGCAGCTGTAAACGCGGCCCTCGTACTCACTATGGCCAAAAAGCTATCCT
    TTTTTAACCAAGCATCCGGAATTCTTTTTTACCGTCGACATTTGCGCCGGGAGCATGAGTGATACCGGTTTTTCGATAGGA


                420           440
GTTCCTGCCACTGCCAGTGAGCTCTGACTAATAGATATCG
    CAAGGACGGTGACGGTCACTCGAGACTGATTATCTATAGCAGCT.
```

which process comprises point mutation of a nucleotide sequence coding for bovine acidic fibroblast growth factor.

2.  A process for the preparation of a plasmid capable of expressing the gene for human acidic fibroblast growth factor which process comprises inserting into a suitable plasmid a nucleotide sequence having the base sequence:

```
1                20                40                60                80
AATTCATGTTCAATCTGCCACCGGGTAATTACAAAAAGCCAAAGCTTCTTTACTGCTCTAACGGTGGTCACTTTCTCCGC
    GTACAAGTTAGACGGTGGCCCATTAATGTTTTTCGGTTTCGAAGAAATGACGAGATTGCCACCAGTGAAAGAGGCG

                100               120               140                 160
 ATCCTGCCAGATGGTACCGTGGACGGCACCAGAGATCGTTCTGATCAACATATTCAACTGCAGCTGTCCGCCGAATCTGT
 TAGGACGGTCTACCATGGCACCTGCCGTGGTCTCTAGCAAGACTAGTTGTATAAGTTGACGTCGACAGGCGGCTTAGACA

                180               200               220               240
 CGGTGAAGTTTACATCAAATCTACCGAAACTGGTCAATACCTTGCCATGGACACTGATGGCCTGCTGTACGGATCCCAGA
  GCCACTTCAAATGTAGTTTAGATGGCTTTGACCAGTTATGGAACGGTACCTGTGACTACCGGACGACATGCCTAGGGTCT

                260               280               300               320
 CCCCAAACGAGGAGTGCCTTTTCCTGGAGCGCCTGGAGGAAAACCATTACAACACCTACATCTCTAAAAAAGCATGCTGAG
  GGGGTTTGCTCCTCACGGAAAAGGACCTCGCGGACCTCCTTTTGGTAATGTTGTGGATGTAGAGATTTTTCGTACGACTC

                340               360               380               400
 AAAAATTGGTTCGTAGGCCTTAAGAAAAATGGCAGCTGTAAACGCGGCCCTCGTACTCACTATGGCCAAAAAGCTATCCT
  TTTTTAACCAAGCATCCGGAATTCTTTTTACCGTCGACATTTGCGCCGGGAGCATGAGTGATACCGGTTTTTCGATAGGA

                420               440
 GTTCCTGCCACTGCCAGTGAGCTCTGACTAATAGATATCG
  CAAGGACGGTGACGGTCACTCGAGACTGATTATCTATAGCAGCT.
```

3. A method of purifying recombinant acidic fibroblast growth factor (aFGF) in pure form comprising the following steps:

a. partial purification of recombinant aFGF by a heparin-Sepharose affinity chromatography matrix and an acceptable eluant; followed by
b. final purification of partially purified recombinant aFGF by reverse phase high performance liquid chromatography using an alkyl silane substrate and an acceptable eluant.

**Patentansprüche**
**Patentansprüche für folgende Vertragsstaaten : AT, BE, CH, DE, FR, GB, IT, LI, LU, NL, SE**

1. Nukleotidsequenz, die für den menschlichen rekombinanten sauren Fibroblasten-Wachstumsfaktor codiert, mit der folgenden Aminosäuresequenz:

```
        1                        10                        20
    PheAsnLeuProProGlyAsnTyrLysLysProLysLeuLeuTyrCysSerAsnGlyGlyHisPheLeuArgIleLeu


           30                        40                        50
    ProAspGlyThrValAspGlyThrArgAspArgSerAspGlnHisIleGlnLeuGlnLeuSerAlaGluSerValGlyGlu


           60                        70                        80
    ValTyrIleLysSerThrGluThrGlyGlnTyrLeuAlaMetAspThrAspGlyLeuLeuTyrGlySerGlnThrProAsn


           90                       100
    GluGluCysLeuPheLeuGluArgLeuGluGluAsnHisTyrAsnThrTyrIleSerLysLysHisAlaGluLysAsnTrp


          110                       120                       130
    PheValGlyLeuLysLysAsnGlySerCysLysArgGlyProArgThrHisTyrGlyGlnLysAlaIleLeuPheLeuPro


          140
    LeuProValSerSerAsp .
```

worin an das Phenylalanin an Position eins ein Methionin angeknüpft ist, worin die Basensequenz lautet:

```
    1            20            40            60            80
AATTCATGTTCAATCTGCCACCGGGTAATTACAAAAAGCCAAAGCTTCTTTACTGCTCTAACGGTGGTCACTTTCTCCGC
        GTACAAGTTAGACGGTGGCCCATTAATGTTTTTCGGTTTCGAAGAAATGACGAGATTGCCACCAGTGAAAGAGGCG


            100           120           140           160
ATCCTGCCAGATGGTACCGTGGACGGCACCAGAGATCGTTCTGATCAACATATTCAACTGCAGCTGTCCGCCGAATCTGT
    TAGGACGGTCTACCATGGCACCTGCCGTGGTCTCTAGCAAGACTAGTTGTATAAGTTGACGTCGACAGGCGGCTTAGACA


            180           200           220           240
CGGTGAAGTTTACATCAAATCTACCGAAACTGGTCAATACCTTGCCATGGACACTGATGGCCTGCTGTACGGATCCCAGA
    GCCACTTCAAATGTAGTTTAGATGGCTTTGACCAGTTATGGAACGGTACCTGTGACTACCGGACGACATGCCTAGGGTCT


            260           280           300           320
CCCCAAACGAGGAGTGCCTTTTCCTGGAGCGCCTGGAGGAAAACCATTACAACACCTACATCTCTAAAAAGCATGCTGAG
    GGGGTTTGCTCCTCACGGAAAAGGACCTCGCGGACCTCCTTTTGGTAATGTTGTGGATGTAGAGATTTTTCGTACGACTC


            340           360           380           400
AAAAATTGGTTCGTAGGCCTTAAGAAAAATGGCAGCTGTAAACGCGGCCCTCGTACTCACTATGGCCAAAAAGCTATCCT
    TTTTTAACCAAGCATCCGGAATTCTTTTTACCGTCGACATTTGCGCCGGGAGCATGAGTGATACCGGTTTTTCGATAGGA


            420           440
GTTCCTGCCACTGCCAGTGAGCTCTGACTAATAGATATCG
    CAAGGACGGTGACGGTCACTCGAGACTGATTATCTATAGCAGCT.
```

2. Expressionsplasmid, umfassend darin insertiert die Nukleotidsequenz nach Anspruch 1.

3. Wirt, der mit dem Plasmid nach Anspruch 2 kompatibel ist und es enthält.

4. Plasmid nach Anspruch 2, das in der Lage ist, das Gen des menschlichen sauren Fibroblasten-Wachstumsfaktors zu exprimieren.

5. Verfahren zur Reinigung des rekombinanten sauren Fibroblasten-Wachstumsfaktors (aFGF) in reiner Form, umfassend die folgenden Stufen:
   a. teilweise Reinigung von rekombinantem aFGF durch eine Heparin-Sepharose-Affinitätschromatographie-matrix und ein akzeptables Elutionsmittel; gefolgt von
   b. einer Endreinigung des teilweise gereinigten rekombinanten aFGF durch reverse phase-Hochleistungsflüssigkeitschromatographie unter Verwendung eines Alkylsilan-Substrats und eines akzeptablen Elutionsmittels.

**Patentansprüche für folgende Vertragsstaaten : ES, GR**

1. Verfahren zu Herstellung einer Nukleotidsequenz, die für den menschlichen rekombinanten sauren Fibroblasten-Wachstumsfaktor codiert, mit der folgenden Aminosäuresequenz:

```
     1                        10                        20
   PheAsnLeuProProGlyAsnTyrLysLysProLysLeuLeuTyrCysSerAsnGlyGlyHisPheLeuArgIleLeu


          30                        40                        50
   ProAspGlyThrValAspGlyThrArgAspArgSerAspGlnHisIleGlnLeuGlnLeuSerAlaGluSerValGlyGlu


                    60                        70                        80
   ValTyrIleLysSerThrGluThrGlyGlnTyrLeuAlaMetAspThrAspGlyLeuLeuTyrGlySerGlnThrProAsn


                    90                       100
   GluGluCysLeuPheLeuGluArgLeuGluGluAsnHisTyrAsnThrTyrIleSerLysLysHisAlaGluLysAsnTrp


          110                       120                       130
   PheValGlyLeuLysLysAsnGlySerCysLysArgGlyProArgThrHisTyrGlyGlnLysAlaIleLeuPheLeuPro


          140
   LeuProValSerSerAsp .
```

worin an das Phenylalanin an Position eins ein Methionin angeknüpft ist, worin die Basensequenz lautet:

27

```
         1              20              40              60              80
AATTCATGTTCAATCTGCCACCGGGTAATTACAAAAAGCCAAAGCTTCTTTACTGCTCTAACGGTGGTCACTTTCTCCGC
        GTACAAGTTAGACGGTGGCCCATTAATGTTTTTCGGTTTCGAAGAAATGACGAGATTGCCACCAGTGAAAGAGGCG


                      100             120             140             160
    ATCCTGCCAGATGGTACCGTGGACGGCACCAGAGATCGTTCTGATCAACATATTCAACTGCAGCTGTCCGCCGAATCTGT·
        TAGGACGGTCTACCATGGCACCTGCCGTGGTCTCTAGCAAGACTAGTTGTATAAGTTGACGTCGACAGGCGGCTTAGACA


                      180             200             ·220            240
CGGTGAAGTTTACATCAAATCTACCGAAACTGGTCAATACCTTGCCATGGACACTGATGGCCTGCTGTACGGATCCCAGA
        GCCACTTCAAATGTAGTTTAGATGGCTTTGACCAGTTATGGAACGGTACCTGTGACTACCGGACGACATGCCTAGGGTCT.


                      260             280             300             320
CCCCAAACGAGGAGTGCCTTTTCCTGGAGCGCCTGGAGGAAAACCATTACAACACCTACATCTCTAAAAAGCATGCTGAG
        GGGGTTTGCTCCTCACGGAAAAGGACCTCGCGGACCTCCTTTTGGTAATGTTGTGGATGTAGAGATTTTTCGTACGACTC


                      340             360             380             400
AAAAATTGGTTCGTAGGCCTTAAGAAAAAATGGCAGCTGTAAACGCGGCCCTCGTACTCACTATGGCCAAAAAGCTATCCT
        TTTTTAACCAAGCATCCGGAATTCTTTTTACCGTCGACATTTGCGCCGGGAGCATGAGTGATACCGGTTTTTCGATAGGA


                      420             440
GTTCCTGCCACTGCCAGTGAGCTCTGACTAATAGATATCG
        CAAGGACGGTGACGGTCACTCGAGACTGATTATCTATAGCAGCT.
```

wobei das Verfahren eine Punktmutation einer Nukleotidsequenz, die für den sauren Rinder-Fibroblastenwachstumsfaktor codiert, umfaßt.

2. Verfahren zu Herstellung eines Plasmids, das in der Lage ist, das Gen des menschlichen sauren Fibroblastenwachstumsfaktor zu exprimieren, wobei das Verfahren umfaßt die Insertion in ein geeignetes Plasmid einer Nukleotidsequenz der folgenden Basensequenz:

AATTCATGTTCAATCTGCCACCGGGTAATTACAAAAAGCCAAAGCTTCTTTACTGCTCTAACGGTGGTCACTTTCTCCGC
GTACAAGTTAGACGGTGGCCCATTAATGTTTTTCGGTTTCGAAGAAATGACGAGATTGCCACCAGTGAAAGAGGCG

ATCCTGCCAGATGGTACCGTGGACGGCACCAGAGATCGTTCTGATCAACATATTCAACTGCAGCTGTCCGCCGAATCTGT·
TAGGACGGTCTACCATGGCACCTGCCGTGGTCTCTAGCAAGACTAGTTGTATAAGTTGACGTCGACAGGCGGCTTAGACA

CGGTGAAGTTTACATCAAATCTACCGAAACTGGTCAATACCTTGCCATGGACACTGATGGCCTGCTGTACGGATCCCAGA
GCCACTTCAAATGTAGTTTAGATGGCTTTGACCAGTTATGGAACGGTACCTGTGACTACCGGACGACATGCCTAGGGTCT

CCCCAAACGAGGAGTGCCTTTTCCTGGAGCGCCTGGAGGAAAACCATTACAACACCTACATCTCTAAAAAAGCATGCTGAG
GGGGTTTGCTCCTCACGGAAAAGGACCTCGCGGACCTCCTTTTGGTAATGTTGTGGATGTAGAGATTTTTCGTACGACTC

AAAAATTGGTTCGTAGGCCTTAAGAAAAATGGCAGCTGTAAACGCGGCCCTCGTACTCACTATGGCCAAAAAGCTATCCT
TTTTTAACCAAGCATCCGGAATTCTTTTTACCGTCGACATTTGCGCCGGGAGCATGAGTGATACCGGTTTTTCGATAGGA

GTTCCTGCCACTGCCAGTGAGCTCTGACTAATAGATATCG
CAAGGACGGTGACGGTCACTCGAGACTGATTATCTATAGCAGCT.

3. Verfahren zur Reinigung des rekombinanten sauren Fibroblasten-Wachstumsfaktors (aFGF) in reiner Form, umfassend die folgenden Stufen:
    a. teilweise Reinigung von rekombinantem aFGF durch eine Heparin-Sepharose-Affinitätschromatographiematrix und ein akzeptables Elutionsmittel; gefolgt von
    b. einer Endreinigung des teilweise gereinigten rekombinaten aFGF durch reverse phase-Hochleistungsflüssigkeitschromatographie unter Verwendung eines Alkylsilan-Substrats und eines akzeptablen Elutionsmittels.

**Revendications**
**Revendications pour les Etats contractants suivants : AT, BE, CH, DE, FR, GB, IT, LI, LU, NL, SE**

1. Séquence nucléotidique codant pour la forme acide, recombinante du facteur de croissance des fibroblastes humain, présentant la séquence d'acides aminés suivante :

1                                    10                                    20

PheAsnLeuProProGlyAsnTyrLysLysProLysLeuLeuTyrCysSerAsnGlyGlyHisPheLeuArgIleLeu

30                                    40                                    50

ProAspGlyThrValAspGlyThrArgAspArgSerAspGlnHisIleGlnLeuGlnLeuSerAlaGluSerValGlyGlu

60                                    70                                    80

ValTyrIleLysSerThrGluThrGlyGlnTyrLeuAlaMetAspThrAspGlyLeuLeuTyrGlySerGlnThrProAsn

90                                    100

GluGluCysLeuPheLeuGluArgLeuGluGluAsnHisTyrAsnThrTyrIleSerLysLysHisAlaGluLysAsnTrp

110                                    120                                    130

PheValGlyLeuLysLysAsnGlySerCysLysArgGlyProArgThrHisTyrGlyGlnLysAlaIleLeuPheLeuPro

140

LeuProValSerSerAsp .

dans laquelle une méthionine est fixée à la phénylalanine sur la première position, la séquence de base étant la suivante :

```
       1              20              40              60              80
AATTCATGTTCAATCTGCCACCGGGTAATTACAAAAAGCCAAAGCTTCTTTACTGCTCTAACGGTGGTCACTTTCTCCGC
    GTACAAGTTAGACGGTGGCCCATTAATGTTTTTCGGTTTCGAAGAAATGACGAGATTGCCACCAGTGAAAGAGGCG


              100             120             140             160
ATCCTGCCAGATGGTACCGTGGACGGCACCAGAGATCGTTCTGATCAACATATTCAACTGCAGCTGTCCGCCGAATCTGT·
    TAGGACGGTCTACCATGGCACCTGCCGTGGTCTCTAGCAAGACTAGTTGTATAAGTTGACGTCGACAGGCGGCTTAGACA


              180             200             ·220            240
CGGTGAAGTTTACATCAAATCTACCGAAACTGGTCAATACCTTGCCATGGACACTGATGGCCTGCTGTACGGATCCCAGA
    GCCACTTCAAATGTAGTTTAGATGGCTTTGACCAGTTATGGAACGGTACCTGTGACTACCGGACGACATGCCTAGGGTCT


              260             280             300             320
CCCCAAACGAGGAGTGCCTTTTCCTGGAGCGCCTGGAGGAAAACCATTACAACACCTACATCTCTAAAAAGCATGCTGAG
    GGGGTTTGCTCCTCACGGAAAAGGACCTCGCGGACCTCCTTTTGGTAATGTTGTGGATGTAGAGATTTTTCGTACGACTC


              340             360             380             400
AAAAATTGGTTCGTAGGCCTTAAGAAAAATGGCAGCTGTAAACGCGGCCCTCGTACTCACTATGGCCAAAAAGCTATCCT
    TTTTTAACCAAGCATCCGGAATTCTTTTTACCGTCGACATTTGCGCCGGGAGCATGAGTGATACCGGTTTTTCGATAGGA


              420             440
GTTCCTGCCACTGCCAGTGAGCTCTGACTAATAGATATCG
    CAAGGACGGTGACGGTCACTCGAGACTGATTATCTATAGCAGCT.
```

2. Plasmide d'expression dans lequel est insérée la séquence nucléotidique selon la revendication 1.

3. Hôte compatible avec le plasmide de la revendication 2 et contenant ce plasmide.

4. Plasmide selon la revendication 2, susceptible d'exprimer le gène de la forme acide du facteur de croissance des fibroblastes humain.

5. Procédé pour purifier la forme acide recombinante du facteur de croissance des fibroblastes (aFGF) sous forme pure, qui comprend les étapes consistant :
   a. à procéder à une purification partielle de l'aFGF recombinant par une matrice de chromatographie d'affinité sur héparine-Sépharose et un milieu acceptable ; puis
   b. à procéder à une purification finale de l'aFGF recombinant partiellement purifié, par chromatographie en phase liquide haute performance en phase inversée, par utilisation d'un substrat d'alkylsilane et d'un éluant acceptable.

**Revendications pour les Etats contractants suivants : ES, GR**

1. Procédé pour préparer une séquence nucléotide codant pour la forme acide, recombinante humaine, du facteur de croissance des fibroblastes, présentant la séquence d'acides aminés suivante :

31

1                                      10                                  20

PheAsnLeuProProGlyAsnTyrLysLysProLysLeuLeuTyrCysSerAsnGlyGlyHisPheLeuArgIleLeu

                  30                                  40                                  50

ProAspGlyThrValAspGlyThrArgAspArgSerAspGlnHisIleGlnLeuGlnLeuSerAlaGluSerValGlyGlu

                  60                                  70                                  80

ValTyrIleLysSerThrGluThrGlyGlnTyrLeuAlaMetAspThrAspGlyLeuLeuTyrGlySerGlnThrProAsn

                  90                                  100

GluGluCysLeuPheLeuGluArgLeuGluGluAsnHisTyrAsnThrTyrIleSerLysLysHisAlaGluLysAsnTrp

                  110                                 120                                 130

PheValGlyLeuLysLysAsnGlySerCysLysArgGlyProArgThrHisTyrGlyGlnLysAlaIleLeuPheLeuPro

                  140

LeuProValSerSerAsp .

dans laquelle une méthionine est fixée à la phénylalanine sur la première position, la séquence de base étant la suivante :

```
      1              20              40              60              80
AATTCATGTTCAATCTGCCACCGGGTAATTACAAAAAGCCAAAGCTTCTTTACTGCTCTAACGGTGGTCACTTTCTCCGC
      GTACAAGTTAGACGGTGGCCCATTAATGTTTTTCGGTTTCGAAGAAATGACGAGATTGCCAGCAGTGAAAGAGGCG


              100             120             140             160
ATCCTGCCAGATGGTACCGTGGACGGCACCAGAGATCGTTCTGATCAACATATTCAACTGCAGCTGTCCGCCGAATCTGT·
TAGGACGGTCTACCATGGCACCTGCCGTGGTCTCTAGCAAGACTAGTTGTATAAGTTGACGTCGACAGGCGGCTTAGACA


              180             200             ·220             240
CGGTGAAGTTTACATCAAATCTACCGAAACTGGTCAATACCTTGCCATGGACACTGATGGCCTGCTGTACGGATCCCAGA
GCCACTTCAAATGTAGTTTAGATGGCTTTGACCAGTTATGGAACGGTACCTGTGACTACCGGACGACATGCCTAGGGTCT


              260             280             300             320
CCCCAAACGAGGAGTGCCTTTTCCTGGAGCGCCTGGAGGAAAACCATTACAACACCTACATCTCTAAAAAGCATGCTGAG
GGGGTTTGCTCCTCACGGAAAAGGACCTCGCGGACCTCCTTTTGGTAATGTTGTGGATGTAGAGATTTTTCGTACGACTC


              340             360             380             400
AAAAATTGGTTCGTAGGCCTTAAGAAAAATGGCAGCTGTAAACGCGGCCCTCGTACTCACTATGGCCAAAAAGCTATCCT
TTTTTAACCAAGCATCCGGAATTCTTTTTACCGTCGACATTTGCGCCGGGAGCATGAGTGATACCGGTTTTTCGATAGGA  ·


              420             440
GTTCCTGCCACTGCCAGTGAGCTCTGACTAATAGATATCG
CAAGGACGGTGACGGTCACTCGAGACTGATTATCTATAGCAGCT.
```

lequel procédé comprend une mutation ponctuelle d'une séquence nucléotide codant pour le facteur de croissance des fibroblastes de boeuf sous forme acide.

2.  Procédé pour préparer un plasmide susceptible d'exprimer le gène de la forme acide humaine du facteur de croissance des fibroblastes, lequel procédé consiste à insérer dans un plasmide approprié une séquence nucléotide ayant la séquence de bases suivante :

```
        1              20             40             60             80
      AATTCATGTTCAATCTGCCACCGGGTAATTACAAAAAGCCAAAGCTTCTTTACTGCTCTAACGGTGGTCACTTTCTCCGC
          GTACAAGTTAGACGGTGGCCCATTAATGTTTTTCGGTTTCGAAGAAATGACGAGATTGCCACCAGTGAAAGAGGCG


                       100            120            140            160
       ATCCTGCCAGATGGTACCGTGGACGGCACCAGAGATCGTTCTGATCAACATATTCAACTGCAGCTGTCCGCCGAATCTGT
          TAGGACGGTCTACCATGGCACCTGCCGTGGTCTCTAGCAAGACTAGTTGTATAAGTTGACGTCGACAGGCGGCTTAGACA


                       180            200           ·220            240
      CGGTGAAGTTTACATCAAATCTACCGAAACTGGTCAATACCTTGCCATGGACACTGATGGCCTGCTGTACGGATCCCAGA
          GCCACTTCAAATGTAGTTTAGATGGCTTTGACCAGTTATGGAACGGTACCTGTGACTACCGGACGACATGCCTAGGGTCT


                       260            280            300            320
      CCCCAAACGAGGAGTGCCTTTTCCTGGAGCGCCTGGAGGAAAACCATTACAACACCTACATCTCTAAAAAGCATGCTGAG
          GGGGTTTGCTCCTCACGGAAAAGGACCTCGCGGACCTCCTTTTGGTAATGTTGTGGATGTAGAGATTTTTCGTACGACTC


                       340            360            380            400
      AAAAATTGGTTCGTAGGCCTTAAGAAAAATGGCAGCTGTAAACGCGGCCCTCGTACTCACTATGGCCAAAAAGCTATCCT
          TTTTTAACCAAGCATCCGGAATTCTTTTTTACCGTCGACATTTGCGCCGGGAGCATGAGTGATACCGGTTTTTCGATAGGA


                       420            440
       GTTCCTGCCACTGCCAGTGAGCTCTGACTAATAGATATCG
          CAAGGACGGTGACGGTCACTCGAGACTGATTATCTATAGCAGCT.
```

3. Procédé pour purifier la forme acide recombinante du facteur de croissance des fibroblastes (aFGF) sous forme pure, qui comprend les étapes consistant :

a. à procéder à une purification partielle de l'aFGF recombinant par une matrice de chromatographie d'affinité sur héparine-Sépharose et un milieu acceptable ; puis

b. à procéder à une purification finale de l'aFGF recombinant partiellement purifié, par chromatographie en phase liquide haute performance en phase inversée, par utilisation d'un substrat d'alkylsilane et d'un éluant acceptable.

# FIG-1